# NEUE EUROPÄISCHE PATENTSCHRIFT

(11) **EP 2 298 254 B2**
(45) Veröffentlichungstag und Bekanntmachung des Hinweises auf die Entscheidung über den Einspruch: **26.06.2024**
(45) Hinweis auf die Patenterteilung: 16.07.2014
(21) Anmeldenummer: 10010955.2
(22) Anmeldetag: 28.05.2004
(51) Int. Cl.: A61F 9/008, A61F 9/01, B23K 26/06, B23K 26/00, A61F 9/009

(54) **Vorrichtung zum präzisen Bearbeiten von Material**
High-precision material processing method and device
Procédé et dispositif de traitement précis d'un materiau

(30) Priorität: 02.06.2003 US 475583 P; 23.07.2003 US 625157
(43) Veröffentlichungstag der Anmeldung: 23.03.2011
(62) Teilanmeldung aus: 04735284.4
(73) Patentinhaber: Carl Zeiss Meditec AG, 07745 Jena (DE)
(72) Erfinder: Bendett, Mark, Ann Arbor MI 48104 (US); Bischoff, Mark, 07749 Jena (DE); Gerlach, Mario, 16548 Glienicke-Nordbahn (DE); Mühlhoff, Dirk, 07751 Jena (DE)
(74) Vertreter: Patentanwälte Geyer, Fehners & Partner mbB

(56) Entgegenhaltungen:
- EP-A1- 0 911 921
- EP-A1- 1 281 378
- WO-A-01/13838
- WO-A-01/54853
- DE-A1- 19 827 139
- US-A- 5 656 186
- US-A- 5 993 438
- US-A1- 2002 003 440
- US-A1- 2002 023 903
- Heisterkamp: "Einsatz ultrakurzer Laserpulse m der refraktiven Laserchirurgie?, Hannover, Univ. Dis., 2002, available to the publicat the lateston October 10, 2002

## Beschreibung

Die Erfindung betrifft ein Femtosekunden-Lasersystem zur präzisen Bearbeitung von Material und Gewebe, insbesondere ein Lasergerät zur präzisen, mikrometergenauen Bearbeitung von organischem Material.

In einem wertvollen Beitrag zum Stand der Technik wird in der Patentschrift DE 197 46 483 der Anmelderin beschrieben, wie mit Mikrometerpräzision bei der großflächigen Bearbeitung von Materialien mit Lasern mit großem Spotdurchmesser (mm - cm) makroskopische Materialmengen ablatiert, verdampft oder geschmolzen werden (CO₂-Laser, Nd:YAG, Excimer ...).

In einem weiteren wertvollen Beitrag zum Stand der Technik wird in der Patentschrift DE 197 27 573 der Anmelderin ein Algorithmus beschrieben, wie ein Laserstrahl abgelenkt werden kann, um eine bestmögliche und präzise Bearbeitung von Material zu gewährleisten.

In der US 5, 656, 186 wird ein Verfahren zum Bearbeiten vom Material bei gleichzeitiger Vermeidung oder Minimierung von schädigenden Nebenwirkungen (Schmelzränder, thermische Schädigung, akustische Schockwellen, Rissbildung) durch Wahl einer speziellen Pulsdauer in Abhängigkeit vom Material beschrieben.

Die materialbearbeitende Wirkung des Lasers ist dabei auf den kleinen Raumbereich des Laserfokus (typischerweise einige µm³) beschränkt, in dem die Lichtintensität hoch genug ist, um die Schwelle des optischen Durchbruchs zu überschreiten. Lokalisiert auf dieses Fokusvolumen wird der Zusammenhalt des Materials zerstört und es entsteht eine Kavitationsblase. Wird der Laserfokus für jeden Laserpuls an eine neue Position gelenkt, können lineare, flächige oder dreidimensionale Schnittmuster generiert werden. Der Abstand benachbarter Kavitationsblasen muss am Ende der Bearbeitung etwa ihrem Durchmesser entsprechen, damit das Material entlang der Schnitte leicht mechanisch ablösbar ist.

Die bestehenden Lasergeräte für die Materialbearbeitung mit Femtosekunden-Laserpulsen verwenden regenerative Verstärker mit Repetitionsraten bis 15 kHz, mit denen einzelne Pulse eines Femtosekundenoszillators verstärkt werden. Während der Oszillator selbst nur Pulsenergien im Nanojoule Bereich bereitstellt, können die Pulse mit einem regenerativen Verstärker bis zu einigen Millijoule Pulsenergie verstärkt werden. Während diese Laserquellen für Anwendungen mit hohen Abtragsraten pro Laserpuls geeignet sind, sind sie nicht optimal für die oben beschriebene Anwendung für Präzisionsschnitte.

Es ist bekannt, solche Laser für die refraktive Hornhautchirurgie zu verwenden. Übliche Pulsenergien betragen 5µJ bis 10µJ. Dadurch werden Kavitationsblasen erzeugt, deren Durchmesser 10µm bis 30µm beträgt. Durch diese Blasengröße wird eine Mikrorauhigkeit des erzeugten Schnittes in gleicher Größenordnung bewirkt. Bekannt ist andererseits, dass eine Mikrorauhigkeit in dieser Größenordnung nur unbefriedigende refraktive Ergebnisse gestattet.

Aus der WO 01/054853 A ist eine Vorrichtung zum präzisen Bearbeiten von Material bekannt, die einen gepulsten Laser aufweist, welcher gepulste Laserstrahlung mit einer Pulslänge zwischen 50 fs und 1 ps und einer Pulsfrequenz von über 100 kHz abgibt. Die Laserbearbeitung erfolgt durch Ablation eines technischen Materials, beispielsweise elektronischer oder optoelektronischer Materialien.

Die US 5993438 A befasst sich mit der refraktiven Laserchirurgie. Dabei wird Laserstrahlung in die Augenhornhaut eingestrahlt und dort zur Wirkung gebracht.

Die US 2002/023903 A1 befasst sich ebenfalls mit der Laserstrahlbearbeitung durch gepulste Laserstrahlung. Dabei kommt ein besonderer Scanner zum Einsatz, der dispersionsfrei arbeitet.

Die WO 03/059563 A2 offenbart die Verwendung gepulster Laserstrahlung zur Augenchirurgie, wobei Werte für Pulslänge, Pulsfrequenz und Pulsdauer der gepulsten Laserstrahlung genannt werden.

Die Veröffentlichung "Medical applications for ultra pulsed laser pulses" von H. Lubatschowski et al., in Rieken Review Nr. 50 (Januar 2003): Focussed on laser position micro fabrication (LPM 2002), Seiten 113-118, schildert die Augenchirurgie mittels gepulster Laserstrahlung und erwähnt bestimmte Werte für Pulsfrequenz, Pulsdauer und Pulswiederholrate.

Ebenfalls mit der Anwendung gepulster Laserstrahlung für die Augenchirurgie befasst sich die US 6325792.

In K. König et al., Optics Letters Vol. 26, No. 11 (2001*)* wurde beschrieben, wie auch mit Nanojoule-Pulsen aus einem Femtosekunden-Oszillator Schnitte in Gewebe ausgeführt werden können. Da dabei aber ein einzelner Laserpuls nicht zur Ausbildung einer Kavitationsblase führt, sondern mehrere, an die gleiche Stelle plazierte Pulse nötig sind, um eine Schnittwirkung zu erzielen, eignet sich dieses Verfahren nur für sehr feine Schnittfiguren im Mikrometermaßstab. Für den industriellen bzw. medizinischen Einsatz ist diese Laserquelle nicht geeignet.

Aufgabe der vorliegenden Erfindung ist es daher, eine Vorrichtung zur präzisen Bearbeitung von Material bereitzustellen, mit der diese Nachteile des Standes der Technik überwunden werden.

Diese Aufgabe wird durch die Vorrichtung gemäß Anspruch 1 gelöst. Weitere vorteilhafte Ausgestaltungen sind in den abhängigen Ansprüchen angegeben.

Insbesondere ist vorgesehen eine Vorrichtung zur präzisen Bearbeitung von Material, insbesondere organischem Material, wobei diese Vorrichtung im zu bearbeitenden Material Kavitationsblasen erzeugt, deren Durchmesser weniger als 10µm beträgt. Um dies zu erreichen, wird ein gepulster Laserstrahl mit einer Pulsenergie von weniger als 5µJ auf einen Fokusdurchmesser von wenigen µm fokussiert. Vorzugsweise beträgt der Fokusdurchmesser etwa 3µm und die Pulsenergie 1µJ. Weiterhin zeichnet sich die Vorrichtung dadurch aus, dass sie durch Verwendung einer Pulswiederholrate von mehr als 50 kHz eine sehr schnelle Bearbeitung gestattet. Dies ist insbesondere für die refraktive Hornhautchirurgie von großem Vorteil, weil damit eine Operationszeit von wenigen Sekunden bis ca. 1 Minute erreicht wird.

Die Aufgabe wird des Weiteren gelöst durch eine Vorrichtung zur präzisen Bearbeitung von Material, nämlich organischem Material, umfassend ein gepulstes Lasersystem mit den oben beschriebenen Parametern als Strahlquelle, bei dem durch eine Strahleinrichtungen mit mindestens einem Mittel zur Strahlablenkung ein Arbeitsstrahl der Strahlquelle auf das Material applizierbar ist, wobei die Pulsaussendung mit der Strahlablenkung korreliert und wobei das Mittel zur Strahlablenkung Mittel zur Freigabe von Laserpulsen umfasst. Unter Freigabe wird dabei verstanden, dass der Laser für einen Laserimpuls freigegeben wird und der Laserimpuls ausgelöst wird, sobald der Laser entsprechend seiner maximalen Repetitionsrate erneut einen Laserimpuls abgeben kann. Unter Korrelation der Pulsaussendung mit der Strahlablenkung wird insbesondere verstanden, dass die Pulsaussendung erfolgen kann, wenn der Strahl auf einen bestimmten Punkt gelenkt wurde, die Pulsaussendung also in Abhängigkeit der Strahlablenkung angesteuert wird.

In einer besonderen Ausgestaltung ist vorgesehen eine Vorrichtung zur präzisen Bearbeitung von Material, insbesondere organischem Material, umfassend ein gepulstes Lasersystem als Strahlquelle, wobei die Energie der Strahlung etwa 100 nJ bis 10 µJ, vorzugsweise 500 nJ bis 5 µJ, beträgt. Die Repetitionsrate der Strahlung beträgt dabei vorzugsweise 50 khz bis 1 Mhz, besonders bevorzugt 100 khz bis 500 khz. Der Fokusdurchmesser der Strahlung beträgt dabei vorzugsweise etwa 500 nm bis 10 µm, besonders bevorzugt 3µm bis 5µm. Die Pulsdauer der Strahlung beträgt vorzugsweise etwa 100 fs bis 1 ps, besonders bevorzugt 200 fs bis 500 fs.

Die Mittel zur Strahlformung und/oder Strahlablenkung bzw. allgemeiner formuliert die Strahlformungs- und -ablenkungssysteme können diffraktive oder refraktive Mikrooptiken oder adaptive Optiken oder klassische optische Systeme umfassen. Mit diffraktiven oder refraktiven Elementen kann man dabei mehrere klassische bzw. konventionelle optische Elemente ersetzen.

Die genannte Vorrichtung zur präzisen Bearbeitung von Material wird eingesetzt für die ophthalmologische Augenbehandlung, insbesondere zur Korrektur der Fehlsichtigkeit eines Auges. Die Vorrichtung kann zum Schneiden eines Flaps oder Lentikels in der Cornea zur Korrektur der Fehlsichtigkeit verwendet werden. Neben einem Schneiden des Lentikels können mit der erfindungsgemäßen Vorrichtung refraktive Strukturen in der Cornea, beispielsweise in Form flächenmäßig nebeneinander gesetzter Spots oder einer Punktwolke, erzeugt werden.

Ebenso können unmittelbar Laserschüsse zur Erzeugung refraktiver Strukturen gesetzt werden. Beispielsweise können in der Augenlinse kleine Bläschen durch verdampfen von Material bzw. Flüssigkeit erzeugt werden. Dazu sind sehr viele Laserschüsse mit vergleichsweise niedriger Energie erforderlich, wie sie mit der erfindungsgemäßen Vorrichtung bereit gestellt werden können.

Ebenso ist es möglich, mit der erfindungsgemäßen Vorrichtung gezielte Schnitte in das Gewebe, beispielsweise der Augenlinse, einzubringen und damit die Krümmbarkeit und Elastizität der Augenlinse zu verbessern, da sich die benachbarten Gewebeteile nun leichter gegeneinander verschieben lassen. Die Vorrichtung zur präzisen Bearbeitung wird in dieser Ausgestaltung der Erfindung als Vorrichtung zur Behandlung der Presbyopie eingesetzt. Die Strahlformung erfolgt entweder konventionell oder mit diffraktiven bzw. refraktiven Mikrooptiken oder adaptiven Optiken. Die Strahlablenkung erfolgt vorzugsweise über Scansysteme.

Die Laserstrahlquellen ist eine Oszillator-Verstärker-Anordnungen, wobei für den Verstärker insbesondere regenerative Verstärker, Chirped-Pulse-Verstärker (CPA) oder Multipass-Verstärker geeignet sind.

Hinsichtlich der Bauform des modengekoppelten Oszillators sind insbesondere Scheibenlaseroszillatoren, Faserlaseroszillatoren, aber auch Stablaseroszillatoren geeignet. Der Verstärker ist ein Faserlaserverstärker. Als Pumpquelle für die Lasermedien sind Halbleiter-Laserdioden aufgrund ihrer langen Lebensdauer, Zuverlässigkeit, Regelbarkeit und ihrer vergleichsweise geringen Herstellungskosten besonders vorzuziehen.

Bevorzugte Lasermedien in obigen Laserstrahlquellen sind dotierte Festkörpermaterialien, insbesondere Kristalle und Gläser. Beispielsweise sind dies YAG, Wolframate, Saphir und Fluoridgläser.

Diese Wirtsmaterialien können bevorzugt mit Neodym, Erbium, Titan, Chrom, Lithium oder Ytterbium dotiert werden. Alle diese Materialien zeichnen sich durch eine spektral breitbandige Laseremission im Spektralbereich von 600 nm bis 2000 nm aus und umfassen damit den für die refraktive Hornhautchirurgie besonders geeigneten Spektralbereich zwischen 800 nm und 1200 nm.

Die große spektrale Bandbreite der Laseremission der oben genannten Materialien unterstützt eine ultrakurze Laserpulsdauer zwischen 50 fs und 1 ps. Dabei ist es nicht erforderlich, dass der Laser selbst Pulse dieser Pulsdauer emittiert, dass aber die bevorzugte Impulsdauer von etwa 300 fs im zu bearbeitenden Werkstück bzw. auf seiner Oberfläche erreicht wird. Zu diesem Zweck umfasst die Vorrichtung ein optisches Modul welches dazu dient, die spektrale Phasenfunktion der Laserpulse geeignet zu beeinflussen. So erzeugt dieses optische Modul einen linearen Pre-Chirp, dessen Betrag dem linearen Chirp des optischen Systems angepasst ist. Diese optische Modul kann bereits in einer Laserstrahlquelle geeignet integriert sein, insbesondere kann es mit dem Pulskompressor einer CPA-Laserstrahlquelle kombiniert oder mit diesem identisch sein.

Das mit Mikrometergenauigkeit zu bearbeitende Material ist organisches Material, nämlich das Gewebe des menschlichen Auges.

Das gepulste Lasersystem ist eine Anordnung einer Laserstrahlquelle zur Erzeugung von fs-Pulsen und entsprechenden optischen Vorrichtungen, insbesondere Spiegel, Linsen, etc.

In einer Ausgestaltung der erfindungsgemäßen Vorrichtung ist vorgesehen, dass das Mittel zur Strahlablenkung im Scan-Modus betrieben werden. Der Arbeitsstrahl der Strahlquelle kann dabei auf in einer Dimension periodisch wiederkehrenden Bahnen abgelenkt werden, sodass beispielsweise kreisförmige Bahnen unterschiedlicher Durchmesser oder spiralförmige Bahnen erzeugt werden können. Die Bahnen des Arbeitsstrahles können durch eine rotierende oder in anderer Weise auf einer Bahn gehaltenen Vorrichtung, beispielsweise durch einen Spiegel, eine Linse, ein Gitter oder dergleichen, erzeugt werden. Die Mittel zur Strahlablenkung können Scanner, z.B. mechanische Scanner, umfassen, die auf vorgegebenen Bahnen bewegbar gelagert sind. Die vorliegende Erfindung nutzt schnelle Ablenksysteme, die den Laser auf den natürlichen Bahnen des Ablenksystems ablenkt, also z.B. auf Kreisbahnen oder Spiralbahnen bei rotierenden Ablenksystemen. Anstatt einzelne Positionen anzufahren und dort einen Laserimpuls auszulösen, sobald die vorgegebene Position erreicht ist und das Ablenksystem wieder ruht, wird die Bahn des Ablenksystems ohne Stops durchlaufen und die Pulse werden durch eine vorgewählte, über die Bahngeschwindigkeit der Fokusbewegung vorgegebene Repetitionsrate beginnend zu einem definierten Zeitpunkt abgegeben.

Sobald also die Fokusposition einen bestimmten Punkt erreicht hat, wird der Laser freigegeben und damit Laserpulse auf das Bearbeitungsgebiet gesendet. Dies führt zu einer Spur von Wirkvolumina, mithin durch den Laserfokus während der kurzen Pulsdauer modifizierte Stellen im Material, entlang einer im wesentlichen vordefinierten Bahn, die insbesondere dadurch ausgezeichnet ist, dass benachbarte Wirkvolumina in gleichbleibendem, vordefiniertem Abstand, beispielsweise in der Größenordnung des Durchmessers der Kavitationsblasen, platziert werden. Durch leichte Modifikation der natürlichen Bahn des Ablenksystems, z.B. durch eine leichte Reduktion des Kreisbahnradius, beispielsweise um den Betrag des Abstandes benachbarter Wirkvolumina, können weitere Spuren geschrieben werden, die sich zu einer Schnittfläche ergänzen. Beispielsweise können hier konzentrische Bahnen oder spiralförmige Bahnen oder dergleichen erzeugt werden. Bei Verwendung eines Ablenkspiegels kann dies beispielsweise durch eine Veränderung der Neigung bei gleichbleibender Rotation des Spiegels geschehen. Ziel ist es, die gewünschte Schnittfläche mit einem gleichmäßigen Raster von Wirkvolumina bzw. Laserfoki zu überdecken. Die natürlichen Bahnen des Ablenksystems können aufgrund der hohen Repetitionsrate des Lasersystems sehr schnell mit definiertem zeitlichem Ablauf durchfahren werden. Die Anpassung der zeitlichen Abfolge der Laserpulse führt dann zur gewünschten Überdeckung der Schnittfläche mit Laserschüssen.

Bei einem weiteren Ausführungsbeispiel der vorliegenden Erfindung sind weiterhin Strahleinrichtungen zur Strahlformung und/oder Strahlführung und/oder Strahlablenkung und/oder Strahlfokusierung vorgesehen. Durch diese Strahleinrichtungen kann der Strahl genau so auf das zu bearbeitende Material gelenkt und geleitet werden, wie es die geplante Anwendung erfordert. Die hier auf einen Fokusdurchmesser in der Größenordnung von 3 µm fokussierten ultrakurzen Laserpulse können insbesondere aufgrund ihrer geringen Pulsenergie von etwa 1µJ in einer kleinen, präzisen Kavitationsblase den Materialzusammenhalt lösen und/oder strukturelle Veränderungen im Material hervorrufen ohne benachbarte Gebiete im Material thermisch, akustisch oder mechanisch zu belasten. Für makroskopische Schnitte und Strukturen im Zentimetermaßstab wird der Laserfokus dreidimensional durch das zu bearbeitende Material gescannt. Der Anwendungsfall bestimmt, wie Strahlquelle, Strahlführung und -formung, Scanner, Scanalgorithmus und Fokussieroptik aufeinander abgestimmt werden, um eine hohe Bearbeitungsgeschwindigkeit bei gleichzeitig hoher Präzision zu erreichen.

Die Strahlformung geschieht dabei bevorzugt mittels eines Teleskops (bevorzugt Galilei-Teleskop mit Sammel- und Streulinse), das den Strahldruchmesser so aufweitet, dass der Laser auf einen entsprechend kleinen Fokus fokussiert werden kann. Bevorzugt wird ein Linsensystem verwendet, das die Abbildungsfehler des Teleskops weitgehend minimiert.

Die Strahlführung erfolgt bevorzugt durch Spiegel oder Spiegelpaare, mit denen der Strahl in die einzelnen Subkomponenten justiert werden kann.

Die Strahlablenkung können konventionelle Scanner bzw. mechanische Laserstrahl-Ablenksysteme wie Galvanometerspiegel im Close-Loop-Betrieb, etc. sein. Bevorzugt jedoch sind mechanische Scanner, die vorgegebene Bahnen (z.B. Kreisbahnen) abfahren und durch Triggerung der Strahlquelle an den vorgesehenen Positionen dadurch Laserpulse ausgelöst werden. So kann auf einem großen Bereich der Schnittfläche mit voller Repetitionsrate bei relativ langsamen Scannerbewegungen gearbeitet werden.

Die Strahlfokussierungseinrichtung dient dazu, im Fokus des Strahls auf oder innerhalb des Materials den Zusammenhalt des Materials aufzuheben (Photodisruption). Im Allgemeinen geht das mit einer lokalen Verdampfung des Materials einher. Bevorzugt wird der Laser hierfür auf einen Durchmesser im Mikrometerbereich fokussiert. Dies liegt nahe am Beugungslimit von Licht im sichtbaren bzw. nahen Infrarotbereich. Die Fokussieroptik weist daher bevorzugt eine hohe numerische Apertur und damit eine kurze Brennweite und eine große optische Öffnung (aufgeweiteter Laserstrahl Durchmesser) aus. Bevorzugt wird der von der Laserquelle ausgehende Strahl vor der Fokussierung auf das Material bzw. Gewebe im Durchmesser aufgeweitet. Die Systeme zur Strahlführung, -ablenkung und -fokussierung sind daher bevorzugt für einen großen Stahldurchmesser ausgelegt.

Laserquelle, Stahlablenkung (Scanner) und Fokussieroptik sind so aufeinander abgestimmt, dass präzise und schnelle Schnittführung im Wege der Fotodisruption ermöglicht wird. Dabei werden Laserspots mit einem Fokusdurchmesser von einigen 100 nm bis einigen µm mit einem Spotabstand in der Größenordnung des Kavitationsblasendurchmessers im Material platziert.

In einer besonders bevorzugten Ausführungsform sind die Strahleinrichtungen, insbesondere die Ablenkeinrichtungen, programmierbar. Durch die Abstimmbarkeit der einzelnen Strahleinrichtungen aufeinander und die Steuerung durch entsprechende Programme kann das System der Strahleinrichtungen zusammen mit dem gepulsten Lasersystem genau auf das Material und die Schnittanforderung eingestellt werden, für die es eingesetzt werden soll. So kann in Abhängigkeit der Transparenz und Brechkraft des zu bearbeitenden Materials sowie der Anforderung an Schnittgeometrie und Operationsdauer das Set an Parametern durch das Programm vorgewählt und abgestimmt werden.

In einer weiteren bevorzugten Ausführungsform der vorliegenden Erfindung sind weiterhin Haltevorrichtungen zur Positionierung und/oder Fixierung des zu bearbeitenden Materials vorgesehen. Durch diese Haltevorrichtungen wird sichergestellt, dass die mikrometergenauen Strukturen, die durch den erfindungsgemäßen Laser hergestellt werden können, nicht durch unkontrollierbare Bewegungen des zu bearbeitenden Materials, insbesondere des menschlichen Auges, beeinträchtigt werden.

Eine solche Fixier- und Positioniervorrichtung kann eine einfache Klemmvorrichtung für ein Werkstück sein, das bevorzugt mit Mehrachsen-Justagemöglichkeiten zur Bewegung und Verkippung des Werkstücks zur optimalen Justage ausgestattet ist. Fixiereinrichtungen für die medizinische Anwendungen an Organen wie zum Beispiel dem Auge müssen außerdem den jeweiligen biologischen Gegebenheiten angepasst sein. Die Fixierung des menschlichen Auges kann zum Beispiel mit Hilfe eines speziellen Adapters und eines Vakuum-Saugringes erfolgen.

Mit den beschriebenen hohen Repetitionsraten kann in Abstimmung mit den beschriebenen Pulsenergien geringen Betrages und der Ablenkeinrichtungen die Laserwirkung für die Photodisruption präzise lokalisiert werden. Hierdurch wird in einem scharf begrenzten Fokusvolumen das Materialgefüge zerstört, in dicht benachbarten Bereichen (von weniger als ein Mikrometer entfernt) findet im Allgemeinen keine Veränderung des Materials statt. Daraus ergibt sich eine hohe Bearbeitungspräzision (Mikrometergenauigkeit) bei Schonung benachbarter Materialregionen. Thermische und mechanische Beanspruchung der nicht bearbeiteten Regionen sind deutlich geringer als bei anderen Bearbeitungsmethoden.

Bei einer weiteren bevorzugten Ausführungsform der erfindungsgemäßen Vorrichtung ist durch die Strahleneinrichtung, insbesondere die Ablenkeinrichtung, ein Arbeitsstrahl der Strahlquelle in geometrisch vorbestimmbarer Form in zeitlich vorbestimmbarem Verlauf auf das Material applizierbar. Durch das Zusammenspiel der einzelnen Komponenten ist so möglich, Schnitte und Strukturierungen zu erzeugen. Zur Erzeugung eines Spots, in dem das Materialgefüge aufgelöst wurde, genügt im Allgemeinen ein Laserpuls mit definiertem Pulsparametern (Pulsenergie, Pulsdauer, Fokus). Für Schnitte und Strukturierung ist eine Vielzahl solcher Spots dicht nebeneinander zu plazieren. Der Abstand benachbarter Spots sollte am Ende der Prozedur in der Größenordnung der Kavitationsblasen liegen. Dafür kann der Laserfokus scannend über bzw. durch das Material bewegt werden. Der Laserfokus folgt in idealer Weise 3-dimensional mit Mikrometergenauigkeit einer vorgegebenen geometrischen Bahn. So ist es beispielsweise möglich, einen Schnitt in dem zu bearbeitenden Material dadurch zu erzeugen, dass eine beliebige Fläche, zum Beispiel eine Rechteckfläche benachbarter Mikrometerspots in dem Gewebe nacheinander scannend angesteuert wird. Dadurch wird genau in dieser Ebene der Materialzusammenhalt aufgelöst und dadurch ein "Schnitt" im Gewebe erzeugt. Genauso ist es möglich, den Laserfokus durch Kreisbewegungen des Scanners in einer Kreisbahn auf das zu bearbeitende Material zu applizieren. Durch eine sich anschließende helixförmige Führung des Bearbeitungsstrahls kann so beispielsweise eine Zylinderfläche aus dem Material herausgeschnitten werden. Da die Photodisruption bevorzugt in einem sehr engen Bereich stattfindet, kann der Laserstrahl auch im Gewebe wirken, ohne dass das vom Laserstrahl außerhalb des Fokus durchdrungene Material beschädigt wird. Auf diese Weise sind beliebige geometrische Bahnen und damit Formen durch Photodisruption in dem Material herausschneidbar.

Bei der refraktiven Hornhautchirurgie kann mit der erfindungsgemäßen Vorrichtung eine spezielle Schnittführung realisiert werden. Dabei wird kein traditioneller Flap präpariert, sondern das zuvor mit der erfindungsgemäßen Vorrichtung in der Cornea präparierte Lentikel über einen oder mehrere begrenzte seitliche Schnitte, welche ebenfalls mit der erfindungsgemäßen Vorrichtung erzeugt werden, am Umfang extrahiert. Zu diesem Zweck kann es vorteilhaft sein, dass Lentikel zuvor durch einen oder mehrere Schnitte mit der erfindungsgemäßen Vorrichtung zu zerteilen. Insbesondere ist eine Zerteilung derart sinnvoll, dass anschließend eine Entfernung der Teile mittels Absaugung mit einer Saug-Spül-Kanüle erfolgen kann.

Beim bevorzugten Ausführungsbeispiel der vorliegenden Erfindung ist eine Vorrichtung vorgesehen, bei der der gepulste Arbeitsstrahl durch die Strahlablenkungseinrichtung auf das Material applizierbar ist und währenddessen die Repetitionsrate der Pulse des Arbeitsstrahles modifizierbar ist. Durch das Vorsehen einer Einrichtung zur Modifizierung der Repetitionsrate bei gleichzeitiger Strahlführung des Arbeitsstrahles über das zu bearbeitende Material kann auf diese Weise elegant ein gleichmäßiges Spotmuster auf dem zu behandelnden Material erzeugt werden, auch wenn der Strahl unter verschiedenen Winkeln bzw. verschieden schnell durch die Ablenkeinrichtung auf das zu bearbeitende Material gerichtet wird. Ein besonders augenfälliger Vorteil wird beispielsweise dann erreicht, wenn die Ablenkeinrichtung den Strahl in Kreisbahnen auf das zu bearbeitende Material lenkt und diese Kreisbahnen mit einer speziellen Umlauffrequenz der Ablenkeinrichtung, insbesondere beispielsweise der Ablenkspiegel, erzeugt wird. Wird bei einer Umlauffrequenz von beispielsweise 50Hz der Laserstrahl auf einer Kreisbahn von 1cm Durchmesser bei einer Repetitionsrate von 300kHz geführt, dann werden auf jeder Kreisbahn pro Umlauf gleichmäßig verteilt 60000 Spots gesetzt. Wenn der Strahl dann auf einem Kreis von nur 0,5cm Durchmesser mit derselben Frequenz der Ablenkeinrichtung geführt wird, kann durch Erniedrigung der Repetitionsrate des gepulsten Arbeitsstrahles der gleiche Abstand der einzelnen Spots voneinander auf dem zu bearbeitenden Material erzeugt werden, wie bei der Strahlführung auf der größeren Kreisbahn. Durch eine entsprechende Modifikation der Repetitionsrate in Abhängigkeit der durch die Ablenkeinrichtung abgefahrenen Geometrie lassen sich so beliebige geometrische Muster mit einem im Wesentlichen gleich bleibenden Spotabstand auf dem zu bearbeitenden Material erzeugen. Beispielsweise können Spiralen abgefahren werden, bei denen von außen nach innen bei gleichbleibender Umlauffrequenz der Ablenkeinrichtung die Repetitionsrate immer weiter abnimmt. Daneben sind auch beliebige andere geometrische Formen denkbar. Ist eine konstante Beabstandung der einzelnen Spots auf dem Material gerade nicht beabsichtigt, sondern soll vielmehr in einem speziellen Bereich eine höhere Spotdichte und in einem weiteren Bereich eine niedrigere Spotdichte erreicht werden, so kann dies ebenfalls durch Kombination der gewählten Parameter für die Repetitionsrate des Arbeitsstrahles und die Frequenz bzw. den örtlichen Verlauf der Ablenkeinrichtung erzeugt werden. So ist es bevorzugt auch möglich, graduell unterschiedliche Bereiche mit verschiedener Fokusdichte zu erzeugen. Beispielsweise kann bei einem Kreis das Zentrum einen sehr niedrigen Fokusabstand aufweisen während der Fokusabstand zum Rand hin immer größer wird.

Die Vorrichtung wird verwendet in einen Verfahren zur Applikation von fs-Pulsen einer Laserstrahlquelle mit oben genannten Eigenschaften, insbesondere hoher Repetitionsrate und geringer Pulsenergie, auf ein Material, in Form des menschlichen Auges, bei dem im Fokus des Laserstrahls das Material mittels Photodisruption bearbeitet wird bzw. dessen Zusammenhalt aufgelöst wird.

Beim der Erfindung wird der gepulste Laserstrahl mittels einer Ablenkeinrichtung auf das zu bearbeitende Material gelenkt und in Abhängigkeit des hierdurch auf dem Material erzeugten Spotmusters die Repetitionsrate der Pulse des Laserstrahls modifiziert. Auf diese Weise kann jedes beliebige Spotmuster und insbesondere jede beliebige Beabstandung der einzelnen Spots voneinander in der gewünschten Geometrie auf dem zu bearbeitenden Material erzeugt werden. Besonders bevorzugt werden die Spotmuster so auf dem zu bearbeitenden Material verteilt, dass die Kavitationsblase jedes einzelnen Spots, die durch Photodisruption entsteht, genau benachbart zu der Kavitationsblase des nächsten Spots gesetzt wird. Auf diese Weise entsteht dann ein gewünschtes Schnittmuster direkt benachbarter Kavitationsblasen. Für spezielle Anwendungsfälle kann es auch gewünscht sein, die Spots noch enger zu setzen. Dies ist beispielsweise dann empfehlenswert, wenn das zu bearbeitende Material sich nach einer gewissen Zeit wieder erneuert und die Ablösung des Materials für eine spezielle Zeit sichergestellt werden soll, bevor beispielsweise der Bohrkern oder ein sonst herausgeschnittenes Stück des zu bearbeitenden Materials entfernt werden kann. Ebenso ist es denkbar, dass die Spots zuerst mit einer größeren Beabstandung gesetzt werden, um in einem folgenden Schritt die Lücken zwischen den Spots zu füllen und dadurch ein gewünschtes Muster von Kavitationsblasen zu bilden.

Die erfindungsgemäße Vorrichtung kann verwendet werden zur refraktiven Chirurgie durch Bearbeitung der Cornea oder der Linse des Auges.

Im Folgenden sollen weitere vorteilhafte Ausgestaltungen der Erfindung an Hand der Zeichnung erläutert werden. Hierbei zeigt
- Fig. 1: zeigt eine schematische Darstellung eines Ausführungsbeispiels eines erfindungsgemäßen Lasers
- Fig. 2: zeigt ein weiteres Ausführungsbeispiel eines erfindungsgemäßen Lasers mit Operationsmikroskop und zu bearbeitendem Auge;
- Fig. 3: zeigt eine schematische Darstellung von einigen Beispielen möglicher Schnittmustern, die mit dem erfindungsgemäßen Lasersystem ausgeführt werden können;
- Fig. 4: zeigt schematisch eine Detailansicht einer Folge von Laserspots auf Kreislinien und
- Fig. 5: zeigt den zeitlichen Verlauf von Folgen von Laserpulsen im und außerhalb des Laserresonators.
- Fig. 6: zeigt die Schnittführung zur Erzeugung eines Lentikels im Schnitt durch die Cornea
- Fig. 7: zeigt den Vorgang der Extrahierung des geschnittenen Lentikels durch einen kleinen seitlichen Schnitt
- Fig. 8: zeigt das geschnittene Lentikel in der Draufsicht der Cornea
- Fig. 9: zeigt eine weitere Form der Schnittführung wobei das Lentikel zerteilt wird und durch zwei seitliche Schnitte extrahiert werden kann.
- Fig. 10: zeigt eine weitere Ausbildung des erfindungsgemäßen Verfahrens wobei die Linse in viele Teile zerteilt wird welche mit einer Saug-Spül-Einrichtung entfernt werden.

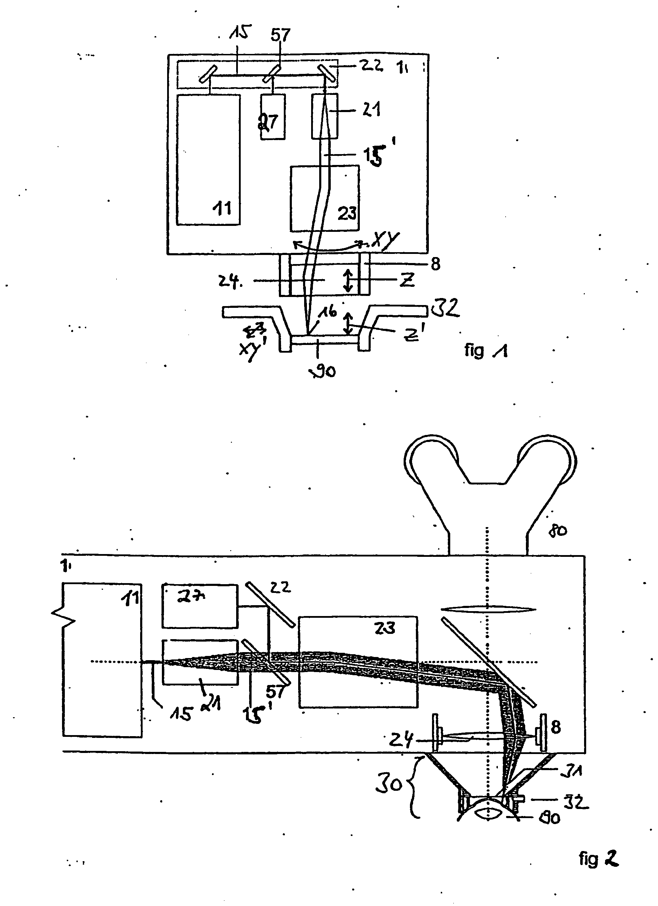

In **Figur 1** ist eine schematische Darstellung der einzelnen Komponenten eines Ausführungsbeispiels eines erfindungsgemäßen Lasersystems dargestellt. Die Bearbeitungsvorrichtung 1 umfaßt als Strahlquelle 11 eine fs-Laserstrahlquelle. Der Laserstrahl 15 wird über Spiegel und einen Strahlteiler 57 auf eine Strahlaufweitungsoptik 21 ausgekoppelt. Der aufgeweitete Laserstrahl 15' wird dann über eine Strahlablenkungseinrichtung wie beispielsweise einen Scanner in XY-Richtung auf eine Strahlfokussierungseinrichtung 24 gelenkt. Diese ist in der Z-Achse verschiebbar und erlaubt so die Verschiebung des Fokuspunktes durch Verschiebung der Strahlfokussierungseinrichtung entlang des Pfeiles Z. Alternativ kann ein fokussierendes optisches System mit veränderlicher Brennweite verwendet werden, um die Fokusposition in Z-Richtung kontrolliert zu verschieben. Der fokussierte Laserspot 16 wird so auf das zu bearbeitende Material 90 gelenkt, das durch eine Fixierungsvorrichtung 32 in seiner Position gehalten wird. Das Material 90 ist hier eine zu bearbeitende Kontaktlinse. Der Spot 16 kann auch durch Verschieben der Fixierungsvorrichtung 32 in Richtung XY' bzw. Z' auf bzw. in dem Material ausgerichtet werden.

Durch die Bearbeitungsvorrichtung 1 wird der von der Strahlquelle 11 erzeugte Laserstrahl 15 auf das Material 90 fokussiert. Ein Fokusdurchmesser von wenigen Mikrometern kann dadurch erreicht werden, dass der Laserstrahl 15 mit einem Strahldurchmesser von einigen Millimetern durch eine Optik mit einigen Zentimetern Brennweite fokussiert wird. Beispielsweise ergibt sich für ein gaußförmiges Strahlprofil ein Fokusdurchmesser von drei Mikrometern, wenn ein Laserstrahl der Wellenlänge 1000 nm und einem Strahldurchmesser von 10 mm mit einer Brennweite von 50 mm fokussiert wird.

Im Allgemeinen besitzt der Laserstrahl 15 am Ausgang der Strahlquelle 11 einen geringeren Strahldurchmesser als zur optimalen Fokussierung notwendig ist. Mit einer Strahlaufweitungsoptik 21 kann der Strahldurchmesser den Erfordernissen angepaßt werden. Bevorzugt kann als Strahlaufweitungsoptik 21 ein auf unendlich eingestelltes Teleskop nach Galilei (Zerstreuungslinse plus Sammellinse) eingesetzt werden. Hierbei entsteht kein Zwischenfokus, der unter Umständen schon zu einem optischen Durchbruch in Luft führen könnte. Damit ist die verbleibende Laserenergie höher und das Strahlprofil gleichbleibend gut. Bevorzugt ist die Verwendung von Linsensystemen, die zu optimalen Abbildungseigenschaften des Teleskops führen. Durch Justage des Teleskops können auch Fertigungsschwankungen in der Strahldivergenz der Strahlquelle 11 ausgeglichen werden.

In diesem Ausführungsbeispiel wird der Laserfokus scannend über bzw. durch das Material bewegt. Der Laserfokus bzw. Laserspot 16 wird so dreidimensional mit Mikrometergenauigkeit gescannt. Der aufgeweitete Laserstrahl 15' wird senkrecht zur ursprünglichen Strahlrichtung durch eine Ablenkeinrichtung 23 abgelenkt. Hierbei verschiebt sich die Lage des Fokus 16 nach der Fokussieroptik 24 senkrecht zur ursprünglichen Strahlrichtung. Damit kann der Fokus in einer Fläche, die im Wesentlichen eben und senkrecht zur Laserstrahlrichtung ist (X/Y-Richtung) bewegt werden. Die Bewegung parallel zur Strahlrichtung (Z-Richtung) kann zum einen durch bewegen des Werkstücks erfolgen (siehe Pfeil Z'). Die Scan-Algorithmen sind dann bevorzugt so ausgelegt, dass das Werkstück nur langsam bewegt werden muß und die schnellen Scannbewegungen von der Ablenkeinheit ausgeführt werden. Zum anderen kann auch die Fokussieroptik parallel zur Laserstrahlrichtung bewegt werden (Pfeil Z), um damit den Fokus in Z-Richtung zu senken. Insbesondere bei medizinischen Applikationen ist die zweite Methode bevorzugt, da der Patient im allgemeinen nicht schnell genug bewegt werden kann.

Das bearbeitete Material 90 wird relativ zum Lasergerät in einer Fixier- und Justagevorrichtung 32 fixiert. Bevorzugt wird hier die Fixiervorrichtung senkrecht und parallel zur Strahlrichtung justiert, um das Schnittmuster an die vorgesehene Stelle im Material 90 plazieren zu können. Ein mit dem bearbeitenden Laserstrahl 15, 15' kolinearer sichtbarer Laserstrahl aus einem Pilotlaser 27 unterstützt hierbei die Justierung.

Zur Strahlführung und zur Feinjustage der Strahllage zwischen den einzelnen Komponenten sind Spiegel bzw. Spiegelpaare 22 vorgesehen. Die Beschaffenheit der Spiegel wird bevorzugt so gewählt, dass der bearbeitende Laserstrahl diesen nicht zerstört, die Spiegel hoch reflektierend für die Wellenlänge des Bearbeitungslasers und hinreichend reflektierend für den Pilotlaser sind. Bevorzugt wird die Beschichtung so gewählt, dass der Spiegel die Laserpulsdauer nicht wesentlich verlängert. Besonders bevorzugt wird mindestens einer der Spiegel ein sogenannter "Chirped Mirror" sein, mit dem die Dispersion aller im Strahlengang vorhandenen Optiken kompensiert werden kann, um optimal kurze Pulse im Bearbeitungsfokus zu erzielen.

In **Figur 2** ist ein weiteres Ausführungsbeispiel der vorliegenden Laserbearbeitungsvorrichtung mit Operationsmikroskop gezeigt. Der Aufbau entspricht im Wesentlichen dem Aufbau in Figur 1. Gleiche Teile sind mit gleichen Bezugszeichen gekennzeichnet. Als Material 90 ist hier ein menschliches Auge vorgesehen. Es soll nun beispielhaft dieses Lasergerät detailiert beschrieben werden, mit dem präzise Schnitte in der Hornhaut des menschlichen Auges eingebracht werden können. Dabei soll eine kreisförmige Fläche, die der Krümmung der Hornhaut folgt und zur optischen Achse des Auges zentriert ist, mit fs-Laserpulsen innerhalb der Hornhaut geschnitten werden. Durch einen kreissegmentförmigen Randschnitt von der Kreisfläche bis zur Außenseite der Hornhaut entsteht ein Hornhautlappen (Flap), der nach dem Laserschnitt zur Seite geklappt werden kann.

Solch ein Flap dient zur Vorbereitung einer LASIK-Operation, bei der durch Laserabtrag die Dicke der Hornhaut so variiert wird, dass refraktive Fehler des Auges kompensiert werden. Bisher wird dieser Schnitt mit einem mechanischen Keratom durchgeführt, was ein hohes Maß an Übung beim Arzt voraussetzt und risikobehaftet ist. Zusätzlich kann durch eine weitere gekrümmte Kreisfläche, die zusammen mit der ersten Kreisfläche des Flaps ein Lentikel umschließt, das nach Aufklappen des Flaps entnommen werden kann, im gleichen Arbeitsgang eine refraktive Korrektur der Hornhaut erfolgen.

Bei der besonderen Ausgestaltung der Erfindung wird das Auge durch einen Saugring 32 an ein Kontaktglas 31 gedrückt, das entweder eben ist, oder bevorzugt der Krümmung der Hornhaut im Wesentlichen angepaßt ist. Der Saugring ist fest mit dem Austrittsfenster des Lasergerätes verbunden, was für eine definierte Lage der Hornhaut relativ zum Laserfokus sorgt. Der aufgeweitete Femtosekunden-Laserstrahl wird mit einer Optik 24 in die Hornhaut fokussiert. Ein Strahlteiler, der für die Laserwellenlänge hochreflektierend und für sichtbares Licht transmitierend ist, spiegelt den Laserstrahl in den Strahlengang eines Operationsmikroskopes ein, das zur Beobachtung und Zentrierung des Auges dient. Die Fokussieroptik 24 bildet dabei einen Teil des Mikroskopobjektives. Zusammen mit einer bündelnden Optik kann ein reelles Zwischenbild der Hornhaut erzeugt werden, das man sich mit dem Stereo-Okular 80 räumlich anschauen kann. Die Strahlablenkeinheit 23 lenkt den aufgeweiteten Laserstrahl 15 senkrecht zu dessen Ausbreitungsrichtung aus. Somit kann der Laserfokus auf unterschiedliche Punkte in der Hornhaut gerichtet werden. Die Fokustiefe kann durch verschieben der Fokussieroptik 24 längs der optischen Achse oder durch Anpassung der Brennweite der Fokussieroptik variiert werden.

Vorzugsweise werden mit der Ablenkeinheit Kreisbahnen abgefahren. Zum Schneiden der Kreisfläche wird der Kreisradius von Kreisbahn zu Kreisbahn verringert und die Repetitionsrate so angepasst, dass ein einheitlicher Spot-Abstand beibehalten wird. Die Fokustiefe wird von Kreisbahn zu Kreisbahn so angepasst, dass der Schnitt der Krümmung der Hornhaut folgt. Sollen astigmatische Korrekturen der Sehkraft (Zylinderkorrektur) eingebracht werden, kann die Fokustiefe während der Kreisbahn zweimal auf und ab bewegt werden, so das ein Lentikel mit Zylinderlinsenanteil entsteht. Für die Flapkante wird bei festem Radius die Fokustiefe vom Flapboden langsam bis zur Außenseite der Hornhaut verschoben, so dass ein Zylindermantel entsteht. Auf einem Bogenstück der dabei beschriebenen Kreise muss der Laserstrahl unterbrochen werden, um einen "Hinge", an dem der präparierte Flap festgehalten wird, zu belassen. Dazu wird einfach das Auskoppeln von Laserpulsen aus der Strahlquelle 11 1 unterbrochen.

Die Strahlquelle 11 ist eine Femtosekunden-Strahlquelle mit den oben beschriebenen Parametern, der bevorzugt direkt diodengepumpt und damit einfach und zuverlässig ist. Der emittierte Laserstrahl 15 wird bevorzugt mit einem Galilei-Teleskop auf 1-2 cm Strahldurchmesser aufgeweitet. Kollinear zum aufgeweiteten Laserstrahl 15 wird ein sichtbarer Laserstrahl aus einem Pilotlaser 27 überlagert, der dann zusammen mit dem Bearbeitungslaserstrahl gescannt und fokussiert wird. Der Strahlteiler 57 ist für diesen Zweck transparent für die Femtosekunden-Laserwellenlänge und reflektierend für den Pilotstrahl.

Die Vielfalt der möglichen Schnittfiguren hängt nur von den Scanalgorithmen ab. Prinzipiell ist ein Lasergerät wie beschrieben zu einer Vielzahl von Applikationen (beispielsweise für refraktive Korrekturen der Sehkraft), bei denen Schnitte oder Strukturumwandlungen innerhalb der transparenten Bestandteile des Auges (Hornhaut, Linse, Glaskörper) und auf den nichttransparenten Teilen wie Sclera, Iris, Zilliarkörper vorgenommen werden sollen, geeignet. Damit übertrifft die Erfindung selbst in diesem kleinen Teilbereich der Anwendung in Universalität und Präzision (Schonung von umliegendem Gewebe) bestehende Technologien bei weitem.

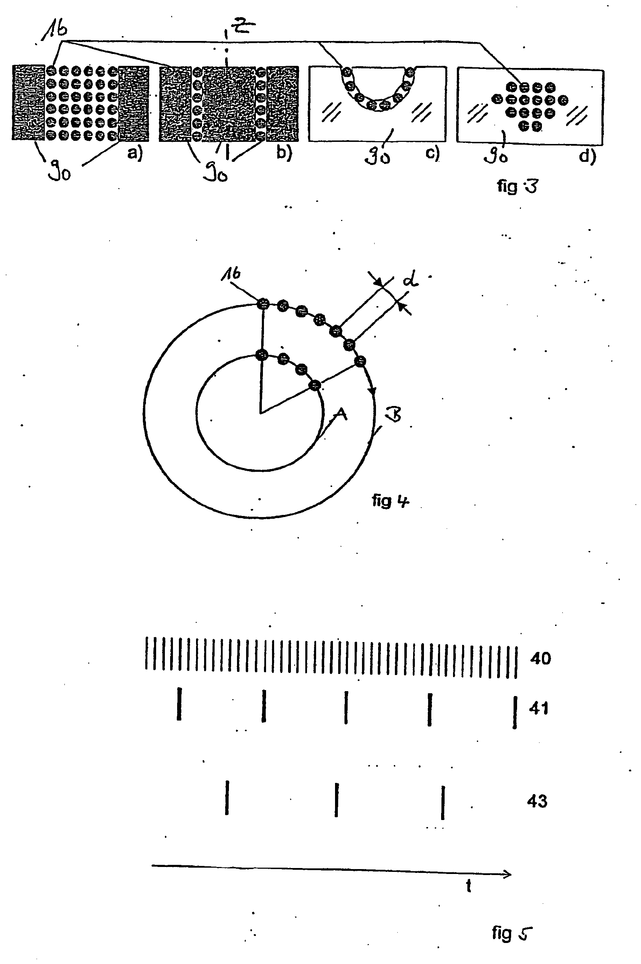

In **Figur 3** sind in den Unterdarstellungen 3 a bis d Anwendungsbeispiele von Schnittgeometrien gezeigt, die mit dem erfindungsgemäßen Lasersystem realisiert werden können. Diese Anwendungen sind nur beispielhaft - es können beliebige weitere Geometrien realisiert werden. Im Fokus 16 des Lasers wird der Zusammenhalt des Materials 90 aufgehoben (Photodisruption). Im Allgemeinen geht das mit einer lokalen Verdampfung des Materials einher. Nach der Einwirkung des Laserpulses ist in einem kleinen Volumen, der Kavitationsblase (im folgenden auch Spot 16 genannt) das Materialgefüge dauerhaft oder für einen bis mindestens zum Ende der Bearbeitungsdauer dauernden Zeitraum aufgehoben. Der Einsatz eines stark fokussierten Femtosekunden-Lasers bietet damit die präziseste Lokalisierung der Laserwirkung. In dem scharf begrenzten Fokusvolumen wird damit das Materialgefüge zerstört, während es in dicht benachbarten Bereichen (schon weniger als ein Mikrometer entfernt) im Allgemeinen keine Veränderung des Materials stattfindet. Daraus ergibt sich eine hohe Bearbeitungspräzision bei Schonung benachbarter Materialregionen.

Für Schnitte und Strukturierungen werden eine Vielzahl von einzelnen Spots, die das Materialgefüge auflösen, dicht nebeneinander platziert. Der Abstand benachbarter Spots sollte am Ende der Prozedur in der Größenordnung des Spotdurchmessers liegen. In Figur 3 a wird ein vorbestimmtes Volumen (z.B. eine Bohrung im Material) durch vollständiges Ausfüllen des abzutragenden Volumens mit einzelnen Spots 16 generiert. Bei einem solchen nicht transparenten Material geht man dabei schichtweise beginnend mit der dem Laser zugewandten Schicht von Spots vor.

In Figur 3b wird nur der Rand der Bohrung mit Spots überdeckt. Es soll hier ein Schnitt durch das Material gezeigt sein. Die Spots 16 sollen rotationssymmetrisch um die gestrichelt eingezeichnete Achse Z angeordnet sein. Auf diese Weise wird ein Bohrkern in der Mitte des bearbeiteten Materials 90 erzeugt. Der Bohrkern kann anschließend als zusammenhängendes Stück entnommen werden. Die benötigte Anzahl von Laserpulsen verringert sich damit insbesondere bei großen Querschnittsflächen der Bohrung erheblich im Vergleich zur Figur 3 a.

In Figur 3c ist eine Unterschneidung in einem transparenten Material 90 gezeigt. Da die Strahlung vom Material 90 nicht absorbiert wird, sind zusammenhängende Materialstücke durch Platzierung von Spots auf der Schnittkante aus dem Material herauslösbar, wenn dieses an die Oberfläche grenzt.

In Figur 3d ist gezeigt, wie in einem transparenten Material je nach Beschaffenheit des Materials Hohlräume bzw. Strukturierungen (z.B. Änderungen der optischen Eigenschaften) erzeugt werden können.

Für makroskopische Schnittfiguren (im Zentimeterbereich) werden einige Millionen Laserspots benötigt, selbst um nur die Schnittfläche (wie in Figuren 3b und c) dicht genug mit Spots zu überdecken. Für viele Anwendungen (insbesondere medizinische Applikationen) ist es vorteilhaft, die Bearbeitungs- bzw. Behandlungszeit so gering wie möglich zu halten. Die Strahlquelle des Lasergeräts ist daher erfindungsgemäß in der Lage, Laserpulse mit einer hohen Repetitionsrate abzugeben. In Figur 4 wird schematisch ein Ausschnitt aus einem möglichen Scanmuster gezeigt, bei dem die einzelnen von einzelnen Laserpulsen bearbeiteten Spots 16 entlang von Bahnen angeordnet sind, die vom Scanner kontinuierlich abgefahren werden können. Um bei hohen Repetitionsraten der Strahlquelle 11 einen hinreichenden großen Spotabstand zu erzielen, wird der Fokus in mindestens einer von drei Scandimensionen sehr schnell bewegt. Die Scan-Algorithmen werden daher bevorzugt so ausgelegt, dass die Spots entlang von Bahnen, die den natürlichen Bewegungen der Ablenkeinheit entsprechen, platziert werden. Die Bewegung in den anderen zwei Dimensionen kann dann relativ langsam erfolgen. Die natürlichen Bahnen der Ablenkeinheit können z.B. Kreisbahnen sein, die die Ablenkeinheiten mit festen Umlauffrequenzen abfahren kann. Das kann z.B. durch rotierende optische Elemente in der Ablenkeinheit erfolgen. Der Radius der Kreisbahn und die Fokustiefe (Z-Richtung) sind dann die langsam veränderbaren Scangrößen. Diese Variante eignet sich besonders, wenn rotationssymmetrische Schnittfiguren erzeugt werden müssen. Die Repetitionsrate des Lasers läßt sich dann besonders effektiv nutzen, wenn die Umlauffrequenz der Kreisbahnen so gewählt wird, dass bei den größten abzufahrenden Kreisbahnen (B) die volle Repetitionsrate der Strahlquelle zum gewünschten Spotabstand d führt. Werden die Kreisbahnen beim Abfahren des Schnittmusters kleiner im Radius (A), kann die Repetitionsrate der Quelle entsprechend verringert werden, so dass sich wieder der optimale Spotabstand ergibt. Diese Anpassung der Repetitionsrate ist bei der beschriebenen Laserstrahlquelle ohne weiteres möglich. Eine Anpassung der Umlauffrequenz an die Repetitionsrate der Quelle kann technologisch schwieriger sein, insbesondere wenn diese kontinuierlich für jede Kreisbahn (A, B) erfolgt. Für eine Verringerung der Bearbeitungszeit kann aber eine Anpassung der Umlauffrequenz in wenigen Schritten an die kleineren Kreisbahnen von Vorteil sein.

In Figur 5 sind mögliche Folgen von Laserpulsen in und außerhalb einer Oszillator-Verstärker-Anordnung dargestellt. Die Umlauffrequenz der Laserpulse im Oszillator 40 hängt nur von der Resonatorlänge ab und ist für eine bestimmte Strahlquelle vorgegeben und liegt bei Resonatorlängen von wenigen Metern um 100 MHz. Bei der hier dargestellten regenerativen Verstärkung werden beispielsweise die Pulse 41 in den Verstärker eingekoppelt und verstärkt. Wird eine geringere Repetitionsrate gewünscht, erfolgt die Verstärkung der Pulse 43. Eine Veränderung der Repetitionsrate der verstärkten Laserimpulse ist auf diese Weise aufwandgering zu realisieren.

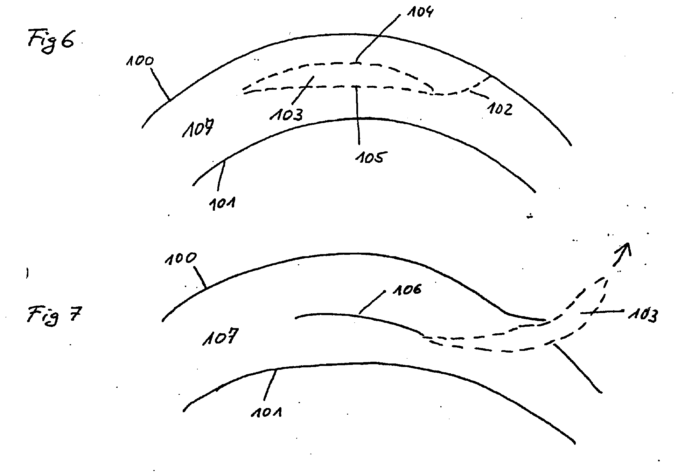

Figur 6 zeigt eine Schnittdarstellung der menschlichen Hornhaut 107 mit Vorderseite 100 und Rückseite 101. Das Lentikel 103 wird durch die beiden flächigen Schnitte 104 und 105 gebildet. Ein kleiner seitlicher Schnitt 102, welcher bis zur Hornhautvorderfläche 100 führt ermöglicht die Extraktion des Lentikels 103. Diese Extraktion ist in Figur 7 dargestellt. Der verbleibende Hohlraum kollabiert 106.

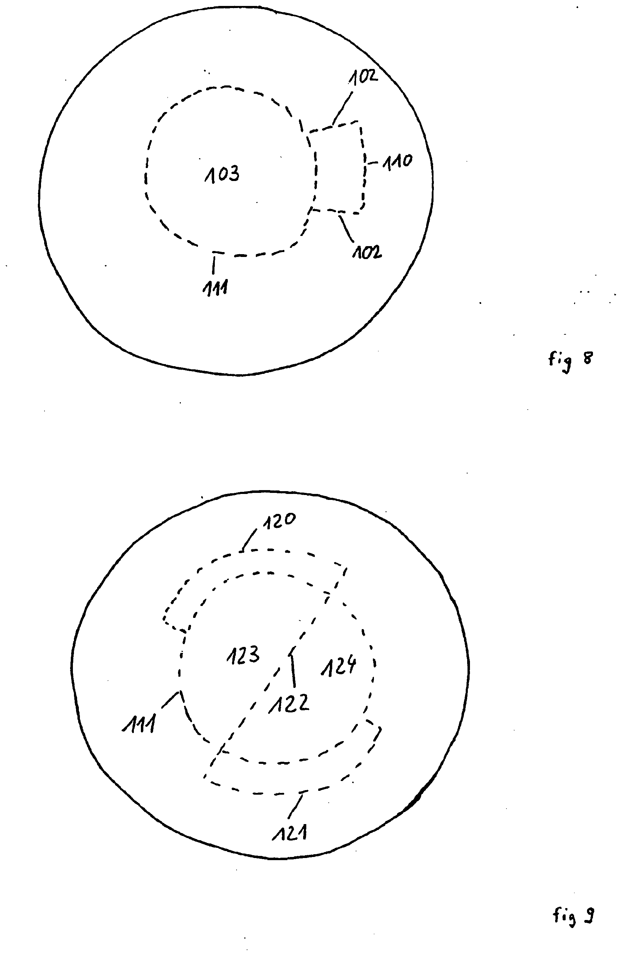

Figur 8 stellt die Hornhaut in der Draufsicht dar. Erkennbar ist die Berandung 111 des Lentikels 103, sowie die an die Hornhautvorderfläche führenden Schnitte 102. Entlang der Linie 110 wird die Hornhautvorderfläche durchtrennt und die Extraktion der Linse ermöglicht.

Figur 9 stellt eine weitere bevorzugte Form der Schnittführung dar. Dabei wurde das Lentikel in zwei Teile 123 und 124 durch einen Schnitt 122 zerteilt. Statt eines einzigen Extraktionsschnittes 110 werden hier zwei Extrakationsschnitte 120 und 121 angebracht. Im nachfolgenden wird das Linsenteil 123 durch den Extraktionsschnitt 120 und das Linsenteil 124 durch den Extraktionsschnitt 121 entfernt.

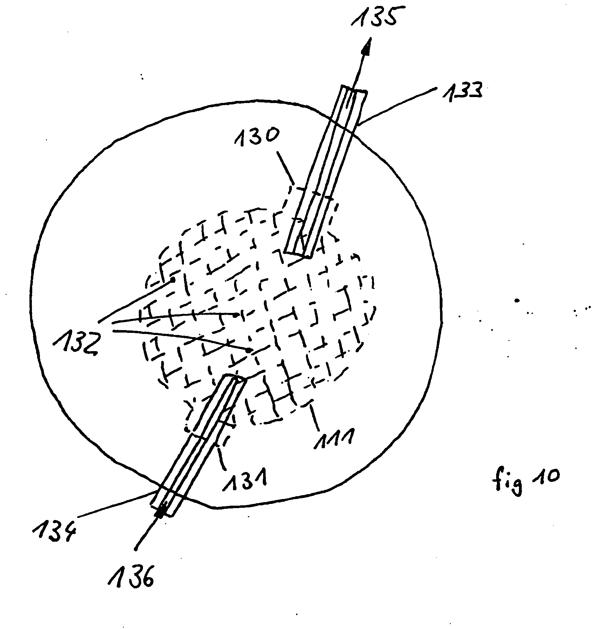

Figur 10 stellt eine weitere Ausprägung des erfindungsgemäßen Verfahrens dar. In dieser Ausprägung wird das durch den Rand 111 berandete Lentikel in viele kleine Fragmente 132 zerschnitten. Diese können nun mit Hilfe einer Kanüle 133, die vorzugsweise einen an die Fragmentgröße angepassten Durchmesser besitzt, abgesaugt werden. Dieser Vorgang kann durch eine Spüleinrichtung über eine zweite Kanüle 134, die in einen gegenüberliegenden Kanal oder auch den gleichen Kanal eingeführt wird, unterstützt werden. Das Spülmittel 136,135 ist vorzugsweise isotonische Kochsalzlösung wobei auch andere Lösungen eingesetzt werden können. Dieses Verfahren realisiert eine minimalste Schwächung der Cornea durch diese Methode der refrakti ven Laserchirurgie.

Die Erfindung wurde an Hand bevorzugter Ausführungsbeispiele erläutert. Fachmännische Weiterentwicklungen führen nicht zu einem Verlassen des durch die Ansprüche definierten Schutzumfangs.

## Patentansprüche

1. Vorrichtung zum präzisen Bearbeiten von Material, nämlich organischem Gewebe in Form der Augenhornhaut oder -linse, mit
- einem gepulsten Laser, der eine Oszillator-Verstärker-Anordnung ist, deren Verstärker ein Faserlaserverstärker ist, und der gepulste Laserstrahlung mit einer Wellenlänge zwischen 600 und 2000 nm und einer Pulsfrequenz von größer als 100 kHz abgibt,
- einem optischen Modul, das eine spektrale Phasenfunktion der Pulse der gepulsten Laserstrahlung beeinflusst und einen linearen Pre-Chirp erzeugt, dessen Betrag einem linearen Chirp eines nachgeordneten optischen Systems angepasst ist,
- eine Strahlfokussierungseinrichtung, welche die der gepulste Laserstrahlung in das Innere des Materials fokussiert,
- wobei die gepulste Laserstrahlung im Material oder auf dessen Oberfläche eine Pulslänge zwischen 50 fs und 1 ps aufweist,
- wobei der gepulste Laser und die Strahlfokussierungseinrichtung so ausgebildet sind, dass die Laserstrahlpulse im innerhalb des Materials gelegenen Fokus eine Photodisruption bewirken.

2. Vorrichtung nach Anspruch 1, wobei die Energie der einzelnen Pulse der Laserstrahlung zwischen 100 nJ und 5 µJ liegt.

3. Vorrichtung nach Anspruch 1 oder 2, wobei weiter Strahleinrichtungen zur Strahlformung und/ oder Strahlführung und/oder Strahlablenkung vorgesehen sind.

4. Vorrichtung nach Anspruch 3, wobei die Strahleinrichtungen programmierbar sind.

5. Vorrichtung nach einem der Ansprüche 1 bis 4, wobei weiter Haltevorrichtungen zur Positionierung und/ oder Fixierung des zu bearbeitenden Materials vorgesehen sind.

6. Vorrichtung nach einem der Ansprüche 3 oder 4, wobei durch die Strahleinrichtungen der Laserstrahl in geometrisch vorbestimmbaren Formen in zeitlich vorbestimmbarem Verlauf in das Material applizierbar ist.

7. Vorrichtung nach Anspruch 6, wobei die Repetitionsrate modifizierbar ist.

8. Vorrichtung nach Anspruch 1, wobei die Oszillator-Verstärker-Anordnung eine Pumpquelle aufweist, die eine Halbleiter-Laserdiode ist.

9. Vorrichtung nach Anspruch 1, wobei die Oszillator-Verstärker-Anordnung einen modengekoppelter Oszillator aufweist, der ein Scheibenlaseroszillator, ein Faserlaseroszillator oder ein Stablaseroszillator ist, die als Lasermedium ein Festkörpermaterial aufweisen, das mit Ytterbium oder Neodym dotiert ist.

10. Vorrichtung nach Anspruch 3, wobei die Strahleinrichtung aufweist:
- eine Ablenkeinrichtung, welche einen Fokus der gepulsten Laserstrahlung in einem Scan-Modus auf einer vorgegebenen Bahn ablenkt, und
- Mittel zur Freigabe, welche am Laser die Abgabe eines der Pulse der Laserstrahlung freigeben,
- wobei die Mittel zur Freigabe ausgebildet sind, die Abgabe eines der Pulse der Laserstrahlung freizugeben, wenn die Ablenkeinrichtung auf eine bestimmte Fokusposition für den Fokus erreicht hat, und damit eine Spur von Wirkvolumina, mithin durch Pulse der Laserstrahlung im Fokus modifizierte Stellen im Material zu erzeugen, wobei entlang der Bahn benachbarte Wirkvolumina in vordefiniertem, gleichbleibendem Abstand liegen.

11. Vorrichtung nach Anspruch 5, wobei die Haltevorrichtung ein an die Augenhornhaut anzudrückenden Kontaktglas (31), dessen Krümmung der der Augenhornhaut angepasst ist, umfasst.

12. Vorrichtung nach Anspruch 1, wobei das optische Modul in den Laser integriert ist.

13. Vorrichtung nach Anspruch 1, wobei die Anpassung derart erfolgt, dass der lineare Pre-Chirp dem Betrag des linearen Chirp des nachgeordneten optischen Systems in Kombination mit dem linearen Chirp der Laserstrahlungsquelle angepasst ist.

## Claims

1. Apparatus for precise processing of material, namely organic tissue in the form of the cornea of an eye, wherein the apparatus comprises:
- a pulsed laser of an oscillator-amplifier setup type, wherein the amplifier is a fiber laser amplifier and wherein the laser provides a pulsed laser beam having a wavelength between 600 and 2000 nm and a pulse frequency of more than 100 kHz,
- an optical module influencing a spectral phase function of the pulses of the pulsed laser beam and generating a linear pre-chirp, wherein the amount of the linear chirp is adapted to a following optical system,
- a beam focusing device focusing the pulsed laser beam into the interior of the material,
- wherein the pulsed laser beam has a pulse length between 50 fs and 1 ps in the material or on its surface,
- wherein the pulsed laser and the beam focusing device are designed such that the laser beam pulses effect photo disruption at a focus located within the material.

2. Apparatus according to claim 1, wherein the energy of the individual pulses of the laser beam is between 100 nJ and 5 µJ.

3. Apparatus according to claim 1 or 2, wherein additional beam devices for forming the beam and/or guiding the beam and/or deflecting the beam are provided.

4. Apparatus according to claim 3, wherein the beam devices are programmable.

5. Apparatus according to any of the claims 1-4, wherein additionally fixtures for positioning and/or fixating the material to be processed are provided.

6. Apparatus according to claim 3 or 4, wherein by means of the beam devices the laser beam pulses are applicable in geometrically pre-determinable shapes and in timely pre-determinable progression into the material.

7. Apparatus according to claim 6, wherein the pulse frequency is modifiable.

8. Apparatus according to claim 1, wherein the oscillator-amplifier setup comprises a pump source in form of a semiconductor laser diode.

9. Apparatus according to claim 1, wherein the oscillator-amplifier setup comprises a mode coupled oscillator, which is a slab laser oscillator, a fiber laser oscillator or a rod laser oscillator, which comprise a solid state material as lasing medium, which material is doted with Ytterbium or Neodym.

10. Apparatus according to claim 3, wherein the beam device comprises:
- a deflecting device which deflects a focus of the pulsed laser beam in a scan mode along a pre-defined path, and
- means for releasing, which release at the laser emission of a pulse of the laser beam,
- wherein the means for releasing are designed to release the emission of a pulse of the laser beam once the deflecting device has reached a certain focus position of the focus and to generate, thus, a path of acted volumina, i.e. locations in the material being modified by pulses of the laser beam at the focus, wherein acted volumina, which are adjacent along the path, are located in a pre-determined constant spacing.

11. Apparatus according to claim 5, wherein the fixture comprises a contact glass (31) to be pressed against the cornea of the eye, wherein the curvature of the contact glass is adapted to the cornea of the eye.

12. Apparatus according to claim 1, wherein the optical module is integrated with the laser.

13. Apparatus according to claim 1, wherein the adaption is thus that the linear pre-chirp is adapted to the amount of the linear chirp of the following optical system in combination with the linear chirp of the laser beam source.

## Revendications

1. Dispositif de traitement précis d'un matériau, à savoir d'un tissu organique sous la forme de la cornée ou du cristallin de l'œil, comprenant :
- un laser pulsé qui est un agencement d'oscillateur et d'amplificateur, dont l'amplificateur est un amplificateur de laser par fibres et émet le rayon laser pulsé à une longueur d'onde comprise entre 600 et 2000 nm et une fréquence d'impulsion supérieure à 100 kHz ;
- un module optique qui influence une fonction de phase spectrale des impulsions du rayon laser pulsé et produit une pré-compression linéaire, dont la valeur est adaptée à une compression linéaire d'un système optique monté en aval ;
- un dispositif de focalisation des rayons qui focalise le rayon laser pulsé à l'intérieur du matériau ;
- étant entendu que le rayon laser pulsé présente une longueur d'impulsion comprise entre 50 fs et 1 ps dans le matériau ou sur sa surface ;
- étant entendu que le laser pulsé et le dispositif de focalisation des rayons sont conçus de telle sorte que les impulsions du rayon laser produisent une photocautérisation dans le point focal situé à l'intérieur du matériau.

2. Dispositif selon la revendication 1, dans lequel l'énergie des impulsions individuelles du rayon laser se situe entre 100 nJ et 5µj.

3. Dispositif selon la revendication 1 ou 2, dans lequel des dispositifs de rayonnement sont également prévus pour la formation de rayons et/ou le guidage de rayons et/ou la déflexion de rayons.

4. Dispositif selon la revendication 3, dans lequel les dispositifs de rayonnement sont programmables.

5. Dispositif selon l'une des revendications 1 à 4, dans lequel des dispositifs de retenue sont également prévus pour le positionnement et/ou la fixation du matériau à traiter.

6. Dispositif selon l'une des revendications 3 ou 4, dans lequel le rayon laser peut être appliqué dans le matériau au moyen des dispositifs de rayonnement dans des formes pouvant être prédéterminées sur le plan géométrique dans une succession pouvant être prédéterminée dans le temps.

7. Dispositif selon la revendication 6, dans lequel le taux de répétition peut être modifié.

8. Dispositif selon la revendication 1, dans lequel l'agencement d'oscillateur et d'amplificateur présente une source de pompage qui est une diode laser à semi-conducteurs.

9. Dispositif selon la revendication 1, dans lequel l'agencement d'oscillateur et d'amplificateur présente un oscillateur à couplage de modes, qui est un oscillateur laser par disque, un oscillateur laser par fibres ou un oscillateur laser par barres, qui présente comme moyen de laser un matériau solide qui est chargé en ytterbium ou en néodyme.

10. Dispositif selon la revendication 3, dans lequel le dispositif de rayonnement présente :
- un dispositif de déflexion qui dévie un point focal du rayon laser pulsé dans un mode de balayage sur un trajet prédéterminé ;
- des moyens de déblocage qui débloquent l'émission d'une des impulsions du rayon laser au niveau du laser ;
- étant entendu que les moyens de déblocage sont conçus pour débloquer l'émission d'une des impulsions du rayon laser lorsque le dispositif de déflexion a atteint une position de focalisation déterminée pour le point focal, et ainsi, pour produire une trace de volumes actifs, et par conséquent, d'endroits modifiés dans le point focal au moyen d'impulsions du rayon laser dans le matériau, étant entendu que les volumes actifs adjacents le long du trajet se situent à une distance constante prédéfinie entre eux.

11. Dispositif selon la revendication 5, dans lequel le dispositif de retenue comprend un verre de contact (31) devant être maintenu en pression sur la cornée, dont la courbure est adaptée à celle de la cornée.

12. Dispositif selon la revendication 1, dans lequel le module optique est intégré dans le laser.

13. Dispositif selon la revendication 1, dans lequel l'adaptation s'effectue de telle sorte que la pré-compression linéaire est adaptée à la valeur de la compression linéaire du système optique monté en aval en combinaison avec la compression linéaire de la source de rayonnement laser.
